# EUROPEAN PATENT APPLICATION

(11) **EP 3 539 657 A1**
(43) Date of publication of application: **18.09.2019**
(21) Application number: 18161655.8
(22) Date of filing: 14.03.2018
(51) Int. Cl.: B01J 35/00, B01J 23/50, C07C 45/37

(54) **IMPROVED CATALYSTS COMPRISING SILVER BASED INTERMETALLIC COMPOUNDS**

(71) Applicant: BASF SE, 67056 Ludwigshafen am Rhein (DE)
(72) Inventor: ZAKZESKI, Joseph John, 67056 Ludwigshafen (DE); LEIDINGER, Peter, 67056 Ludwigshafen (DE); MISRA, Sumohan, 67056 Ludwigshafen (DE); PARVULESCU, Elena, 67056 Ludwigshafen (DE); BOSCH, Marco, 67056 Ludwigshafen (DE); PFEIFFER, Daniel, 67056 Ludwigshafen (DE); WEBER, Susanne, 67056 Ludwigshafen (DE)
(74) Representative: BASF IP Association

(57) **Abstract**

The present invention relates to improved silver-containing catalysts, particularly suitable for the production of aldehydes and/or ketones through the oxidative dehydration of alcohols, especially the oxidative dehydration of methanol to formaldehyde. More specifically, the invention relates to fixed bed catalysts comprising at least one silver based intermetallic compound which is composed of silver and of zirconium or aluminum. Such catalysts exhibit a particularly low increase of the pressure drop during the operation.

## Description

The present invention relates to improved silver-containing catalysts, particularly suitable for the production of aldehydes and/or ketones through the oxidative dehydration of alcohols, especially the oxidative dehydration of methanol to formaldehyde. More specifically, the invention relates to fixed bed catalysts comprising at least one silver (Ag) based intermetallic compound having the general formula AgₘMₙ, where M is a metal from the group consisting of zirconium (Zr) and aluminum (Al). In addition, the present invention provides a corresponding process for the preparation of aldehydes and/or ketones, especially formaldehyde, through the oxidative dehydration of the alcohol using the fixed bed catalyst described in this invention. The present invention also provides a process for preparing such fixed bed catalysts.

The term oxidative dehydration involves the conversion of alcohols to the corresponding aldehyde and/or ketone in the presence of an oxygen containing gas mixture, preferably oxygen, through which at least a part of the hydrogen formed during the course of the reaction is converted to water through oxidation with oxygen. The reaction can take place in the gas phase as well as in the liquid phase, where the gas phase is preferred.

The terms bimetallic, alloy, intermetallic, intermetallic compounds and intermetallic phases are well described in literature (e.g. S. Furukawa and T. Komatsu, ACS Catal. (2017), vol. 7, pages 735-765). Alloys are classified into two categories depending on their structure: solid-solution alloys and intermetallic compounds. The former, typically substitutional solid-solution alloys, consist of metals of similar atomic size and electronic character with a crystal structure identical to that of the parent metal with random atomic arrangements. If the atoms of one element is sufficiently small to fit within the lattice void of the counterpart element, interstitial solid-solution alloys can be formed. The latter involves the opposite situation, where the component metals have significantly different characters and comprise distinct crystal structures with highly ordered atomic arrangements. Intermetallic are defined as a solid containing two or more metals with the intermetallic exhibiting a different crystal structure compared to the two or more metals. Depending on the employed metals, such intermetallic occur at fixed molar ratios of the metals ("line phases") or within a continuous range of ratios.

The production of formaldehyde through the oxidation/dehydration of methanol on a silver catalyst is well-established (Ullmann's Encyclopedia of Industrial Chemistry, Weinheim, 2016, A. W. Franz, H. Kronemayer, D. Pfeiffer, R. D. Pilz, G. Reuss, W. Disteldorf, A. O. Gamer, A. Hilt, Chapter "Formaldehyde", pages 1 - 34; M. Qian, M. A. Liauw, G. Emig, Applied Catalysis A: General, vol. 238 (2003), pages 211-222, "Formaldehyde synthesis from methanol over silver catalysts"; H. Sperber, Chemie Ingenieur Technik, vol. 41 (1969), pages 962-966 "Herstellung von Formaldehyd aus Methanol in der BASF"; A. Schlunke, Dissertation, "Mechanism and Modelling of the Partial Oxidation of Methanol over Silver", University of Sydney, 2007, pages 14-17).

In standard industrial applications, electrolytic silver crystals are produced for use as the catalyst. Silver is oxidized to silver ions at the anode and again reduced at the cathode to form silver particles. The crystalline silver produced on the cathode is the material typically used as the catalyst for the synthesis of formaldehyde form methanol. Especially good catalyst performance was obtained as fixed bed catalysts described in DE 2322757 A. Suitable silver crystals for use as a catalyst can be obtained as described in DE 1166171 B through electrolysis, preferably using a silver nitrate solution dissolved in water. This silver nitrate solution had a pH range preferably between 1 and 4 and contained 1 to 5 weight percent silver. The pH of the solution was controlled with nitric acid. During the electrolysis, silver is typically used as the electrodes. Silver sheets are typically used as the cathode. The electrolysis is preferably conducted with a current density between 80 and 500 A/m² based on the cathode surface and an electrolyte temperature between 10 and 30°C. In order to reach the preferred current density, most of the electrolytic cells are run at an electrical potential from 1 to 15 V. It is advisable to remove the silver crystals formed at the cathode continuously from the surface of the cathode.

Silver crystals with a particle size from 0.2 to 5 mm are typically obtained by the electrolysis. Normally useful crystals are obtained after a single electrolysis cycle. In general, the silver crystals are ordered as fixed beds consisting of 1 to 9 layers of silver crystals (so-called "short layers") with a total layer thickness of 1 to 10 cm.

Two variations for the production of formaldehyde through partial oxidation/dehydration of methanol using silver catalysts are conducted industrially. The BASF-method (the "full conversion" method) involves conversion of more than 96% of the methanol. The remaining unconverted methanol remains in the product mixture since the proportion of methanol in the product is low, obviating additional purification. In this method, methanol is not separated and recycled back to the reactor. In an alternative method, between 77 and 87% of the methanol is converted, necessitating separation and recycling of unconverted methanol. Typical formaldehyde yields for the BASF-method are between 89.5 and 90.5%, whereas the yields of the alternative method are typically between 91 and 92%.

The use of silver crystals as catalysts for the oxidative dehydration of methanol to formaldehyde is known. The production of formaldehyde through oxidative dehydration of methanol by dosing a gas mixture of methanol, oxygen, nitrous oxide to a silver catalyst fixed bed is disclosed in EP 624565. EP 880493 describes a method to produce formaldehyde through the oxidative dehydration of methanol using a gas mixture containing methanol, oxygen, nitrous oxide, and water at a temperature between 150 and 800°C through a phosphorous-doped silver catalyst fixed bed. The pressure drop increases during the course of the reaction as a consequence of inter-crystal silver transformations compress the catalyst bed, which has a negative effect on the catalyst activity (H. Sperber, Chemie Ingenieur Technik, vol. 41 (1969), pages 962-966, "Herstellung von Formaldehyd aus Methanol in der BASF"). It was reported that silver nets form together under reaction conditions and form "dendritic" structures, which leads to pressure drops increasing so such an extent that continuing the manufacturing process is no longer appropriate (Catalyst Manufacture, 2nd Edition (1995), Marcel Dekker, Inc., A. B. Stiles, T. A. Koch, vol. 20, pages 203, "Oxidation of Methanol to Formaldehyde").

Analysis of the reaction process as well as spent silver catalysts have shown that the increase in pressure drop is associated with the formation of a spongy silver structure.

The formation of sponge-like or dendritic structures occurs under reaction conditions and significantly increases the silver surface area available for the reaction. The formation of sponge-like structures does not stop during the production process, but rather leads to always finer-distributed silver filaments and reduced open space in the sponge-structure. The structural changes that occur to at least a portion of the silver crystals, that typically consist of electrolytic silver having 0.2 to 5 mm particle size, to the sponge-like structure explains the observation that conversion and selectivity of freshly started catalysts tends to increase during the start of operation. On the other hand, the increasingly finer sponge-structure results in an increase pressure drop in the reactor, which contributes to unselective gas-phase reactions and results in a loss of selectivity. Accordingly, in the process to produce formaldehyde, an increase in the conversion factor (defined as the tons of methanol needed to produce one ton of formaldehyde, based on 100% formaldehyde concentration) is observed. Similar observations occur when the silver catalyst is used in other forms known from the literature, such as in fibers, nets, extrudates, or foams. In addition to the yield losses, an increased pressure drop contributes to higher operating costs for the compression of the reaction gas. Eventually, the pressure drop increases to the point that it reaches the technical limits of the reactor system to compress the gas, at which point the reactor must be stopped and the catalyst system exchanged. Frequent catalyst exchanges reduce the production capacity of the production system and add additional costs for catalyst removal, catalyst reintroduction, and silver recovery. For silver crystal catalysts, a typical operating time between catalyst exchanges is between 0.5 and 4 months.

US 4,076,754 discloses a two-stage method for production of formaldehyde from methanol, air, and water using a catalyst comprising 40 layers of silver nets with a mesh width of 1.25 mm ("20 mesh silver gauze"), which were produced from silver thread with a diameter of 0.014 inches (350 µm).

DE 2829035 describes a catalyst consisting of catalytically active metal fibers consisting of silver, platinum, rhodium, palladium or a combination of these metals, in which the metal fibers are in the form of needle-composite materials joined together in a felt-like manner. The catalyst can be used for ammonium oxidation or formaldehyde production.

DE 3047193 describes a silver catalyst or a silver alloy in which the catalyst is produced via a melt-spin or melt-extraction technique. Bands of silver between 1 to 2 mm in width and 50 to 60 µm in thickness are shrunk and cut into approximately 1 cm long short-fiber, wavy strips.

EP 0218124 describes a method to produce supported catalysts with a silver layer as active component on carrier material, where the carrier material takes the form of a net, web, or foil. The silver comprises a polycrystalline layer formed through deposition of metallic silver on the carrier and then subsequent thermal treatment from 200 to 800°C.

WO 2012/146528 describes a method to produce C1-C10 aldehydes through oxidative dehydration of C1-C10 alcohols on a catalyst obtained through three-dimensional deformation and/or reordering of silver-containing fibers and/or threads such that the average quadratic cross-section of the silver containing fibers or threads is in a range between 30 and 200 µm. The description also contains the use of a combination of silver-containing fibers and/or threads and silver crystalline particles as the catalyst.

WO 2015/086703 discloses a method to produce metal-foam catalyst, where the metal M(x) consists either as a pure substance or as a mixture with a metal M(y), where the metals M(x) and M(y) are from the group consisting of nickel, chromium, cobalt, copper and silver, including the use of such metal-foam catalyst to produce formaldehyde from methanol.

US 3,959,383 describes a two-step method, where in the first step a silver catalyst derived from fibrous electrolytic silver, crystalline electrolytic silver, or silver on a carrier such as for example aluminum oxide is used, and in the second step a crystalline electrolytic silver catalysts is used. The two-step method is characterized such that the first and second steps are each operated in separated reactors under adiabatic conditions between 575 and 650°C and 600 to 700°C for the first and second reactor, respectively, and that the temperature of the reaction gas leaving the first reactor is actively cooled to <300°C using a heat exchanger before additional oxygen is dosed to the gas stream entering the second reactor.

US 5,973,210 describes the use of intermetallic ruthenium-tin compounds for the catalytic preparation of aldehydes by hydrogenation of the corresponding carboxylic compounds.

It is an objective of the present invention to provide an improved silver based fixed bed catalyst for the production of C1-C10 aldehydes and/or ketones through the oxidative dehydration of the corresponding alcohols, especially the oxidative dehydration of methanol to formaldehyde, and to provide a corresponding production method with conditions that minimizes the pressure drop, or rather that minimizes the rate of pressure drop increase, while least equivalent selectivity and conversion is achieved. The maximum operating time, or rather the time between catalysts exchanges, will be extended through the improved properties of the fixed bed catalyst, the amount of non-selective side reaction taking place in the gas phase reduced, and the operating costs of the production facility minimized as a result of lower compression costs of the inlet gases. Under reaction conditions such that at least equivalent activity and/or selectivity is achieved, the pressure drop or rate of pressure drop increase should be less than that obtained with conventional silver-crystal catalysts.

The objective is achieved through the application of the inventive fixed bed catalysts and inventive method for the production of aldehydes and/or ketones through the oxidative dehydration of the corresponding alcohol on such fixed bed catalyst.

The inventive fixed bed catalyst comprises a layer A which comprises a catalytic component A1 comprising at least one silver (Ag) based intermetallic compound composed of the components Ag and M, where M is a metal from the group consisting of zirconium (Zr) and aluminum (Al).

The silver based intermetallic compound of the invention is composed of the components Ag and M, where M is a metal from the group consisting of zirconium (Zr) and aluminum (Al). Intermetallic compounds of Ag and Zr exist with the fixed compositions AgZr and AgZr₂, and intermetallic compounds of Ag and Al exist within the continuous composition range from Ag_{3.88}Al to Ag_{1.39}Al. Preferably, the silver based intermetallic compound of the invention is from the group consisting of AgZr, Ag₃Al, and AgZr₂. Particularly preferred are the silver based intermetallic compounds AgZr and Ag₃Al.

Preferably, the content of silver based intermetallic compound of the invention in weight percent in the catalytic component A1 is ≥ 30%, more preferably ≥50%, more preferably ≥ 80%, more preferably ≥ 90%, more preferably ≥ 95%, and most preferably ≥ 99%. Typical admixtures to the silver based intermetallic compounds in the catalytic component A1 are silver, zirconium, aluminum or non-intermetallic alloys thereof. In a particular embodiment of the invention, the catalytic component A1 consists of at least one silver based intermetallic compound of the invention. In a further particular embodiment of the invention, the catalytic component A1 consists of one silver based intermetallic compound of the invention.

In a particular embodiment of the invention, the fixed bed catalyst comprises a layer A which consists of the catalytic component A1.

In a particular embodiment of the invention, the fixed bed catalyst comprises a layer A which comprises the catalytic component A1 and at least one additional catalytic component A2 which comprises silver or non-intermetallic alloys thereof. In a further particular embodiment of the invention, the fixed bed catalyst comprises a layer A which consists of the catalytic component A1 and at least one additional catalytic component A2 which comprises silver or non-intermetallic alloys thereof. Preferably, the catalytic component A2 exhibits a content of silver and silver based non-intermetallic alloys (in weight percent) of ≥ 30%, more preferably ≥50%, more preferably ≥ 80%, more preferably ≥ 90%, more preferably ≥ 95%, and most preferably ≥ 99%. More preferably, the catalytic component A2 exhibits a content of silver (in weight percent) of ≥ 30%, more preferably ≥50%, more preferably in a purity ≥ 80%, more preferably ≥ 90%, more preferably ≥ 95%, and most preferably ≥ 99%. In a particular embodiment of the invention, the catalytic component A2 consists of silver or non-intermetallic alloys thereof. In a further particular embodiment of the invention, the catalytic component A2 consists of silver.

The various catalytic components of layer A (e.g. one catalytic component A1 consisting of a silver based intermetallic compound of the invention and one catalytic component A2 consisting of silver) are physical mixtures (e.g.: mixtures of granulates of catalytic component A1 and of catalytic component A2; nets or wads composed of fibers of catalytic component A1 and fibers of catalytic component A2). Usually the ratio of catalytic component A1 to catalytic component A2 is in the range from 10:1 to 1:10, preferably in the range from 5:1 to 1:5, and particularly in the range from 3:1 to 1:3.

The various catalytical components of layer A (e.g. one catalytic component A1 consisting of a silver based intermetallic compound of the invention and one catalytic component A2 consisting of silver) are preferably present in a form selected from the group consisting of nets, wads, and granulates, and particular preferably present in form of granulates.

The nets and wads of the catalytic components of layer A consist of wires in form of fibers or threads, more preferably from fibers or threads with a preferably circular cross-section. The fibers or threads have a length generally in a range from 1 mm to 100 mm, however the inventive silver-containing fibers could be theoretically endless with a practical length ranging from a 1 centimeter to multiple kilometers. The fibers or threads have a wire diameter generally in the range from 30 µm to 200 µm, preferably in the range from 30 µm to 150 µm, more preferably in the range from 30 µm to 100 µm, and most preferably in the range from 30 µm to 70 µm. The three-dimensional deformation and/or restructuring of such fibers or threads can occur in an ordered (nets) or unordered (wads) manner.

The unordered deformation of the inventive silver containing fibers or threads leads to an essentially irregularly structures (wads). Such wads can be pressed to get structures with the desired density or void fraction.
The ordered deformation of the inventive silver containing fibers or threads leads to an essentially regular and ordered structure with periodic repetition of elementary cells, for example stitches or holes. A well-suited method for the orderly shaping and/or rearrangement of the silver containing fibers or preferably silver containing threads in the tangling, weaving or the like, optionally with subsequent compaction. Usually such nets have a mesh size in the range from 300 to 50 mesh (which corresponds to 80 to 500 µm), preferably in the range from 300 to 100 mesh (which corresponds to 80 to 250 µm).

In the context of this invention, the term "net" is broadly understood as any essentially regular or ordered structure of wires (fibers or threads), including knits and gauze.

The granulates of the catalytic components of layer A have an average particle size preferably in the range from 0.3 mm to 3 mm, more preferably in the range from 0.4 mm to 2 mm, and most preferably in the range from 0.5 and 1 mm.

Usually, layer A has a thickness in the range of 1 to 20 mm, preferably in the range of 1 to 4 mm.

In a particular embodiment of the invention, the fixed bed catalyst consists of a layer A.

In a particular embodiment of the invention, the fixed bed catalyst comprises a layer A and an additional layer B which is located downstream to layer A and which comprises a catalytic component B1 consisting of silver (Ag) based granulate, net or wad, preferably of silver (Ag) based granulate. In a preferred embodiment of the invention, such layer B consists of one or more catalytic component B1 consisting of silver (Ag) based granulate, net or wad, preferably of silver (Ag) based granulate. Usually the average particle size of this granulate is in the range from 0.5 mm to 5 mm, preferably in the range from 0.75 mm to 4 mm, and most preferably in the range from 1 and 3 mm. The content of electrolytic silver in weight percent in such catalytic component B1 is usually ≥ 30%, more preferably ≥50%, more preferably in a purity ≥ 90%, more preferably ≥ 99.9%, and most preferably ≥ 99.99%. From a practical perspective, it is advantageous that such layer B has a height of at least 10 mm, more preferably at least15 mm, and most preferably at least 20 mm in order to obtain a homogeneous distribution of catalyst across the cross-section of the reactor. In one embodiment of the invention, layer B is distributed across the reactor with the goal to obtain an equal flow of reaction gas across the entire catalysts bed in order to achieve optimal usage of the catalyst material. In a particular embodiment, layer A and layer B are directly in contact.

The layers A and B can be composed of different sub-layers, such that the form of the catalytic components exhibits different properties. For example, in the form of the use of granulates, sub-layers with different particle sizes, or for example in the case of nets, sub-layers with different mesh widths. In a preferred form of the invention, the layers A and B are built without sub-layers.

In a typical example, the fixed bed catalyst is supported on a carrier. Such carriers are known to one skilled in the art and consist of, for example, grates, baskets or perforated plates, or stabile nets made from a variety of materials including, for example, stainless steel or silver.

According to the invention, the granulates consist of small, generally irregularly-formed solid particles in which the granulates.

The density of inventive silver containing granulates, nets or wads, or arrangements thereof is generally in the range from 1 g/cm³ to 4 g/cm³.The density generally corresponds to a void fraction in the inventive silver containing granulates, nets or wads, or arrangements thereof ranging from 60% to 90%. Such a void fraction of the silver containing granulates, nets or wads, or arrangements thereof is also advantageous to ensure an "ignition" of the catalytic oxidation/dehydrogenation of the alcohols (e.g. methanol) at very low temperatures, for example, 350 °C or less and advantageously in the range from 200 to 350 °C. Typically, the silver containing granulates, nets or wads, or arrangements thereof are preheated until the reaction (e.g. oxidative dehydrogenation of methanol to formaldehyde) lights off. Thereafter, the reaction mentioned is generally self-sustaining under adiabatic conditions. A void fraction of more than 80% is disadvantageous.

The silver based intermetallic compound of the invention can be prepared by any method known in the art. For example, the silver based intermetallic compound can be prepared using an arc melting system equipped with a water-cooled copper hearth. Stoichiometric quantities of high-purity Ag and metal M can be loaded into the copper hearth under a flowing argon atmosphere. A zirconium getter was first melted to remove residual oxygen and then stoichiometric quantities of Ag and metal M can be arc-melted using a tungsten electrode for approximately 20-30 seconds at 35-40 A. The resulting sample can be re-melted additional times and turned over to ensure homogeneity. After the final melt, the obtained intermetallic button can be cooled, crushed, abraded, and sieved to a desired particle size. The formation of an intermetallic compound can be confirmed by means of x-ray diffraction (XRD).

In the inventive silver containing fixed bed catalysts, activation of the employed catalytic components is achieved within 30 days, more preferably within 15 days, and most preferably within 5 days under reaction conditions. Alternatively, the employed catalytic components are activated under particular activation conditions in order to shorten the activation time, preferably to 2 days, more preferably to 1 day or even less. The inventive silver containing fixed bed catalysts allows for an excellent activity and selectivity for the production of aldehydes and/or ketones through the oxidative dehydration of alcohols, while simultaneously providing a minimal increase in pressure drop across the catalyst bed. Without being bound to the theory, the intermetallic based catalyst of the invention is less prone to sinter processes which are a major cause for the increase of the pressure drop.

Under reaction conditions in the context of this invention is to be understood as introduction of the reactant stream, comprising one or more C1-C10 alcohols and one or more other oxidative agents, through the fixed bed catalyst at a temperature in the range from 350°C to 750°C, a gas hourly space velocity in the range from 36,000 h⁻¹ to 1,800,000 h⁻¹ at a gas velocity in the range from 0.1 m s⁻¹ to 15 m s⁻¹. Preferentially the oxidative agent is contained in the gas stream in a composition in the range from 0.01 to 9 wt.% with respect to the complete reactant stream. In a preferential variant, the reactant gas contains an inert gas such as nitrogen and/or additives such as halogenated hydrocarbons including, for example, C₂H₄Cl₂ or C₂CH₂Cl₂, through which the additives are dosed in the ppm-range (for example, maximum 500 wt.-ppm) with respect to the reactant stream.

Under activation conditions in the context of this invention is to be understood as introduction of a gas mixture comprising oxygen and/or one or more other oxidative agents and a material capable of being oxidized (proton donor), such as for example an alcohol or hydrogen, through the fixed bed catalyst at a temperature in the range from 350°C to 750°C, a gas hourly space velocity in the range from 36,000 h⁻¹ to 1,800,000 h⁻¹ at a gas velocity in the range from 0.1 m s⁻¹to 15 m s⁻¹. Preferentially the oxidative agent is contained in the gas stream in a composition between 0.01 and 9 wt.% with respect to the complete gas mixture. In one particular example, hydrogen as a material capable of being oxidized and air as an oxidative material are used in a volume ratio of 1:25. In a preferential variant, the gas mixture contains an inert gas such as nitrogen and/or additives such as halogenated hydrocarbons including, for example, C₂H₄Cl₂ or C₂CH₂Cl₂, through which the additives are dosed in the ppm-range (for example, maximum 500 wt.-ppm) with respect to the gas mixture.

An embodiment of the present invention is also a method of preparing an activate fixed bed catalyst, whereas the fixed bed catalyst is exposed to reaction conditions or activation conditions, preferably for at least 1 day, more preferably at least 3 days, most preferably at least 5 days.

An embodiment of the present invention is also an activated fixed bed catalyst obtained over the course of an activation period under reaction or activation conditions, preferably for at least 1 day, more preferably at least 3 days, most preferably at least 5 days under activation or reaction conditions.

An object of the present invention is also to provide a method to produce C1-C10 aldehydes and/or ketones through the oxidative dehydration of the corresponding C1-C10 alcohols such that the reactant stream, comprising one or more C1-C10 alcohols and one or more oxidative agents at a temperature in the range from 350 to 750°C, more preferentially from 400 to 750°C, more preferentially from 450 to 750°C, more preferentially from 550 to 750°C, and most preferentially in the range from 595 to 710°C, is led through the fixed bed catalyst of the invention.

In a preferential application of the invention, the C1-C10 alcohol consists of methanol, the C1-C10 aldehyde consists of formaldehyde, and the reactant stream is led through the fixed bed catalyst of the invention at a temperature in the range from 550 to 750°C, more explicitly in the range from 595 to 710°C. Under such conditions it is advantageous to produce formaldehyde at full conversion, which requires a high selectivity and simultaneously a high catalyst activity.

The inventive method is preferentially conducted in a reactor. The reactor is preferentially oriented perpendicular to the ground and the reactant stream is typically introduced to the reactor from the top to the bottom of the reactor. Such reactors and systems are described in EP 467169 A, DE 2444586 A und EP 150436 A. Typically, the cross-sectional area of the reactor and the fixed bed catalyst are the same.

In a typical example of the invention, a reactant stream flows through one fixed bed catalyst of the invention (reaction zone).

In one example of the inventive production method, a reactant stream flows into multiple fixed bed catalysts (reaction zones) connected in series, wherein at least one fixed bed catalyst is one of the invention. The reaction zones can be situated in a single reactor or in a reactor cascade.

The gas hourly space velocity for the inventive production method, or rather for the inventive reaction and activation conditions, is typically in the range from 36,000 h⁻¹ to 1,800,000 h⁻¹, preferably in the range from 50,000 h⁻¹ to 1,000,000 h⁻¹, more preferably in the range from 60,000 h⁻¹ to 500,000 h⁻¹, and most preferably in the range from 70,000 h⁻¹ to 300,000 h⁻¹. The gas hourly space velocity (GHSV) with units h⁻¹ is defined as the quotient of the volumetric flow that flows through the fixed bed catalyst (under ambient conditions, p = 1 bar, T = 273,15 K), und the volume of the fixed bed catalyst in the reactor.

The average gas velocity for the inventive production method, or for the inventive reaction and activation conditions, usually lies in the range from 0.1 m s⁻¹ to 15 m s⁻¹, more preferably from0.2 m s⁻¹ to 5 m s⁻¹, more preferably from 0.3 m s⁻¹ to 3 m s⁻¹, and most preferably in the range from 0.5 m s⁻¹ to 2 m s⁻¹. The average gas velocity with the unit m s⁻¹ is defined as the quotient of the volumetric flow that flows through the fixed bed catalyst under normal conditions (p = 1 bar, T = 273.15 K), and the cross-sectional area of the fixed bed catalyst.

The pressure drop over the fixed bed catalyst (or with multiple fixed bed catalysts connected in parallel or in series), measured under reaction conditions, lies typically in a range from 10 mbar to 700 mbar, preferably from 20 mbar to 500 mbar, most preferably from 30 mbar to 300 mbar for the inventive method. The pressure drop increase under reaction conditions under similar residence times is typically in a range from 0.01 mbar per day to 10 mbar per day, more preferably from 0.01 mbar per day to 3 mbar per day, and most preferably from 0.01 mbar per day to 1 mbar per day.

The reactants are preferably in the gas form.

The production is preferably carried out continuously.

The C1-C10 alcohols for the inventive production method or rather for the inventive reaction conditions include alcohols with 1 to 10 carbon atoms and one or more, preferably one to three OH-groups. The alcohols contain preferably one or two OH-groups, most preferably one OH-group. The alcohols contain at least one secondary or primary OH-group, preferably at least one primary alcohol group. The alcohol can be aliphatic, linear, branched, or cyclic, or contain multiple C-C double or triple bonds in the molecule. The alcohol can be an aliphatic or aralkyl alcohol, preferably an aliphatic alcohol.

The C1-C10 alcohols are preferentially chosen from a group consisting of methanol, ethanol, 1-propanol, iso-propanol, n-butanol, iso-butanol, sec.-butanol, 1,2-ethandiol (ethylene glycol), 1,2-propandiol, 1,3-propandiol, 2-(2-hydroxethoxy)ethanol (diethylene glycol), allyl alcohol, 3-methly-2-butenol (prenol) and 3-methyl-3-butenol (iso-prenol). The preferred alcohol is methanol.

The aldehydes or ketones obtained using this invention can contain one or more aldehyde groups in the molecule, preferably one or two aldehyde groups, especially one aldehyde group in the molecule. Examples for the inventive C1-C10 aldehydes include formaldehyde (methanal), glyoxal (ethandial), 2-hydroxyethanal (glycolaldehyde), 2-(2-hydroxyethoxy)ethanol, 3-methyl-2-butenal (prenal), and 3-methyl-3-butenal (iso-prenal).

For the inventive production method, or for the inventive reaction and activation conditions, pure oxygen or an oxygen-containing gas mixture such as, for example, air, or other oxidative gases including nitrogen oxide (NO), nitrogen dioxide (NO₂), dinitrogen oxide (N₂O), dinitrogen tetraoxide (N₂O₄), or a mixture thereof, can be used as oxidative agents for the inventive production method.

In one particular embodiment of the inventive method, methanol is converted to formaldehyde (methanal). Suitable feedstocks include pure methanol, technical methanol, raw methanol produced by a high- or low-pressure production method, or preferably a mixture of methanol with water. The mass component of the methanol in such a water mixture is preferably in the range from 30 to 99 wt.%, more preferably from 45 to 97 wt.%, more preferably from 60 to 95 wt.%, and most preferably from 70 to 90 wt.%. In one variation of the inventive production method, raw methanol, obtained as described in DE 1277834 B, DE 1235881 C or DE 136318 C through separation of a low-boiling fraction or through cleansing with an oxidant or purification with an alkaline, can be used.

In the production method of formaldehyde, oxygen and methanol are usually used in a molar ratio in the range from 0.1:1 to 1.0:1, preferably from 0.25:1 to 0.6:1, most preferably from 0.35:1 to 0.5:1.

In the inventive production method of formaldehyde, methanol is dosed to the reactor zone preferentially in vapor form, preferably as a mixture with steam and/or with an inert gas. The inert gas can be, for example, nitrogen, in which the ratio of the inert gas in the mixture can vary and lies typically in the range from 0.1 to 30 vol.%, preferably in the range from 0,15 to 20 vol.%, more preferably in the range from 0.2 to 10 vol.%, and most preferably in the range from 0.3 to 5 vol.%.

The reaction gas mix in the inventive production method of formaldehyde is generally dosed into the reactor in a temperature range from 50 to 200°C and typically with an absolute pressure in the range from 0.5 to 2 bar, preferably from 1.0 to 1.7 bar. It is advantageous for the inventive method that the gas leaving the reaction zone is cooled to a temperature in the range from 50 to 350°C within a few seconds, for example, within a maximum of 10 seconds. In the inventive production method of formaldehyde, the gas mixture is usually led to an absorptions tower, preferably in counter flow, in which formaldehyde is washed with water or a water-formaldehyde-urea solution. Special variations of this generally known method that can be utilized in the inventive method are described in DE 2444586 A, DE 2451990 A, EP 83427 A and EP 150436 A.

The inventive fixed bed catalysts and inventive production method enables a reduced rate of pressure drop increase relative to conventional catalysts systems and methods with equal or better yield and selectivity during the oxidative dehydration of C1-C10 alcohols to the respective aldehydes and ketones, especially the oxidative dehydration of methanol to formaldehyde. The inventive fixed bed catalyst and production method therefore enable longer production times between catalyst exchanges and simultaneously allows improved yield and selectivity for oxidative dehydration with lower required catalyst mass required for the reaction.

For the nets and wads of the invention, the wire diameter is the average diameter (in the case of an essentially circular cross section) or the average diagonal length (in the case of essentially rectangular or square cross section) of the wires (fibers or threads) that make up the nets or wads. The average diameter of the silver containing fibers or threads (in the case of essentially circular cross section) is determined using the method of DIN ISO 4782 (Oct. 1993, "Metal wire for industrial wire screens") by the means of a stirrup bolt. The average diagonal length of the silver containing fibers or threads (in the case of essentially rectangular or square cross section) is determined analogously (measurement of height and width of the wire and calculation of the diagonal length) also by means of a stirrup bolt.

For the granulate of the invention, the average particle size is determined using the method of DIN 66165-1 and 66165-2 (Aug. 2016, "Particle size analysis - sieve analysis").

For the nets of the invention, the mesh size is determined using the method of DIN ISO 9044 (Aug. 1999, "Industrial woven wire cloth").

The density and the void fraction of the silver containing granulate, nets or wads, or arrangements thereof is the bulk density, which is determined with the method as follows: A body of known geometry is weighed. The ratio of its weight to the volume occupied by it determines the density. The ratio to the weight of a geometrically identical massive body composed of the same material defines the void fraction.

### Figures

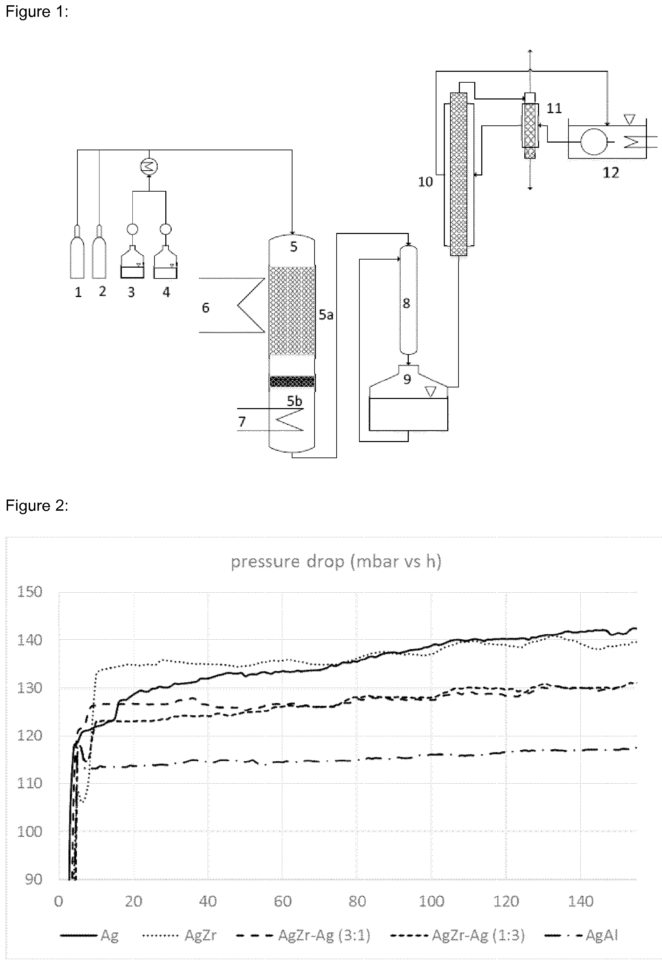

Figure 1: Schematic representation of the exemplary test reactor. The reactant air (1), nitrogen (2), deionized water (3) and methanol (4) are introduced to a reactor column (5) with a preheating section (5a), catalyst packing (5b), electrical heating (6) (not used in the examples of the invention), and temperature quench (7). The product mixture is lead to an absorption column (8) and then from the bottom of the absorption column to a second absorption column (9) equipped with a condenser (10). Finally, the product is purified in a phase separator (11) with cryostat (12).
Figure 2: Pressure drop across the fixed bed catalyst (in mbar; y-axis) versus operating time of the catalyst (in h; x-axis) for a silver based reference catalyst ("Ag" - Comparative Example) and various catalyst which are based on silver containing intermetallic compound of the invention ("AgZr" - Example 1; "AgZr-Ag (3:1)" - Example 2; "AgZr-Ag (1:3)" - Example 3; "AgAI" - Example 4). All these fixed bed catalysts were composed of a thin upper layer (granulate of the characteristic material with a particle size of 0.5 to 1 mm) and thicker bottom layer (granulates of electrolytic silver with a particle size of 1 to 2 mm).

### Examples

Various fixed bed catalyst of the invention (Example 1 to 4) and a comparative catalyst (Comparative Example) were tested. The experiments were conducted in a quartz glass reactor with an inner diameter of 20 mm filled with catalyst. The setting is depicted in Figure 1. The reaction was conducted with a gas-phase water/methanol mixture (molar ratio of water/methanol: 1.0), air (408 NI/h), and nitrogen (150 Nl/h) such that the molar methanol to oxygen ratio of 2.5 is reached. This gas mixture was preheated to a temperature of 150°C before leading the gas stream to the reactor, which was heated externally to 550°C.

When the dosing and preheating temperature is as described above, the catalyst bed typically reaches an ignition reaction temperature in the range from 590°C to 710°C. The gas hourly space velocity falls typically in a range from 85,000 to 120,000 h⁻¹. The product mixture leaving the reactor bed was directly cooled with a heat exchange to 120°C. The composition of the reactant and product mixtures were determined using gas chromatography.

The methanol conversion is defined as the molar amount of converted methanol divided by the molar amount of dosed methanol. The formaldehyde selectivity is defined as the molar amount of obtained in the product stream divided by the molar amount of converted methanol. The consumption figure, a measure for the selectivity of the reaction of methanol to formaldehyde at a given conversion, is defined as the amount of methanol in kilograms that must be dosed to the reactor system in order to obtain 1.00 kilogram of formaldehyde. Activation of the catalyst on one hand and rising pressure drop on the other hand causes the consumption figure to reach an optimum (minimum). In the following examples and comparative examples, the average consumption figure, the methanol conversion and the formaldehyde selectivity were determined as average over a 100 h period after 50 h of operation. Furthermore, the pressure drop across the fixed bed catalyst was monitored during the initial 160 h (Figure 2). The pressure drop increase was determined as average over a 100 h period after 50 h of operation. During this time period the increase of the pressure drop show an essentially linear behavior. The results are summarized in Table 1.

In order to achieve the ignition needed to bring the catalyst into operation, a methanol/water/air/nitrogen mixture was warmed to 300°C, where at this temperature the molar ratio of methanol to oxygen was 7:1 and the nitrogen dosing was 300 NI/h. The ignition occurs at a temperature between 300°C and 350°C. Thereafter the reactant composition was altered in steps to match the composition described above.

The temperature is measured using temperature sensors that are distributed across the cross-section of the catalyst layer. The pressure difference is determined using pressure sensors situated before and after the reactor. The catalyst temperature was controlled by adjusting the air flow to the reactor.

### Comparative Example: Two-layer catalyst with granulates from electrolytic silver

The reactor was filled with a two-layered fixed bed catalyst. The bottom layer consisted of granulate from electrolytic silver with a particle size of 1 to 2 mm with an average thickness of 20 mm. The purity of the silver was 99.99% and the density of the layer was 1.5 g/cm³. The upper layer consisted of granulates made from electrolytic silver with a particle size between 0.5 and 1 mm and with an average thickness of 1 mm. The purity of the silver was 99.99% and the density of this layer was 2.0 g/cm³.

### Example 1: Two-layer catalyst with granulates from AgZr intermetallic compound (upper layer) and granulates from electrolytic silver (bottom layer)

The reactor was filled with a two-layered fixed bed catalyst. The bottom layer was prepared as in the Comparative Example. The upper layer consisted of granulates made from intermetallic AgZr with a particle size of 0.5 to 1 mm and with an average thickness of 1 mm. The AgZr intermetallic compound was prepared using an arc melting system equipped with a water-cooled copper hearth. Stoichiometric quantities of high-purity Ag and Zr were loaded into the copper hearth under a flowing argon atmosphere. A zirconium getter was first melted to remove residual oxygen and then stoichiometric quantities of Ag and Zr were arc-melted using a tungsten electrode for approximately 20-30 seconds at 35-40 A. The resulting sample was re-melted six times and turned over to ensure homogeneity. After the final melt, the obtained intermetallic button was cooled, crushed, and sieved to a particle size of 0.5 to 1 mm. The formation of the intermetallic compound of the formula AgZr was confirmed by XRD.

### Example 2: Two-layer catalyst with a mixture (weight ratio 3:1) of granulate from AgZr intermetallic compound and granulate from electrolytic silver (upper layer) and granulate from electrolytic silver (bottom layer)

The reactor was filled with a two-layered fixed bed catalyst as described for Example 1, but using as upper layer a physical mixture (weight ratio of 3:1) of granulate from AgZr intermetallic compound and granulate from electrolytic silver, whereas both granulates had a particle size of 0.5 to 1 mm and the layer had an average thickness of 1 mm. The granulate from the intermetallic AgZr was prepared as described for Example 1.

### Example 3: Two-layer catalyst with a mixture (weight ratio 1:3) of granulates from AgZr intermetallic compound and granulates from electrolytic silver (upper layer) and granulates from electrolytic silver (bottom layer)

The reactor was filled with a two-layered fixed bed catalyst as described for Example 1, but using as upper layer a physical mixture (weight ratio of 1:3) of granulate from AgZr intermetallic compound and granulate from electrolytic silver, whereas both granulates had a particle size of 0.5 to 1 mm and the layer had an average thickness of 1 mm. The granulate from the intermetallic AgZr was prepared as described for Example 1.

### Example 4: Two-layer catalyst with granulates of Ag₃Al intermetallic compound (upper layer) and granulates from electrolytic silver (bottom layer)

The reactor was filled with a two-layered fixed bed catalyst as described for Example 1, but using intermetallic Ag₃Al instead of intermetallic AgZr. The particle size of the granulate and the thickness of the layer was the same in Example 1. The Ag₃Al intermetallic compound was prepared using an arc melting system equipped with a water-cooled copper hearth. 1 mol of high-purity Al and 3 mol of high-purity Ag were loaded into the copper hearth under a flowing argon atmosphere. A zirconium getter was first melted to remove residual oxygen and then stoichiometric quantities of Ag and Al were arc-melted using a tungsten electrode for approximately 20-30 seconds at 35-40 A. The resulting sample was re-melted six times and turned over to ensure homogeneity. After the final melt, the obtained intermetallic button was cooled, crushed, and sieved to a particle size of 0.5 to 1 mm. The formation of the intermetallic compound (δ-phase) with a nominal composition of Ag₃Al was confirmed by XRD.

**Table 1: Performance of the various silver containing fixed bed catalyst in the oxidative dehydration of methanol to formaldehyde**

| | dP | ΔdP | CF | Conv. | Select. |
|---|---|---|---|---|---|
| | [mbar] | [mbar/100h] | [kg/kg] | [%] | [%] |
| Comp. Example | 138 | 10.45 | 1.23 ± 0.02 | 94.9 ± 2.4 | 91.5 ± 0.9 |
| Example 1 | 138 | 5.49 | 1.32 ± 0.06 | 91.3 ± 3.0 | 89.1 ± 0.9 |
| Example 2 | 128 | 4.40 | 1.25 ± 0.02 | 93.7 ± 1.4 | 91.1 ± 0.6 |
| Example 3 | 128 | 5.52 | 1.23 ± 0.02 | 94.2 ± 1.3 | 92.4 ± 0.5 |
| Example 4 | 116 | 3.27 | 1.21 ± 0.01 | 94.7 ± 1.5 | 93.0 ± 0.7 |

Comp. Example (upper layer: granulate of Ag with 0.5 to 1 mm particle size; lower layer: granulate of Ag with 1 to 2 mm particle size), Example 1 (upper layer: granulate of AgZr intermetallic compound with 0.5 to 1 mm particle size; lower layer: granulate of Ag with 1 to 2 mm particle size), Example 2 (upper layer: physical mixture of granulate of AgZr intermetallic compound and granulate of Ag (ratio 3:1 b.w.) both with 0.5 to 1 mm particle size; lower layer: granulate of Ag with 1 to 2 mm particle size); Example 3 (upper layer: physical mixture of granulate of AgZr intermetallic compound and granulate of Ag (ratio 1:3 b.w.) both with 0.5 to 1 mm particle size; lower layer: granulate of Ag with 1 to 2 mm particle size); Example 4 (upper layer: granulate of Ag₃Al intermetallic compound with 0.5 to 1 mm particle size; lower layer: granulate of Ag with 1 to 2 mm particle size); dP (average pressure drop across the fixed bed catalyst during 100 h starting 50 after operation start, measured in mbar); ΔdP (increase of pressure drop during 100 h starting 50 after operation start, measured in mbar/100h by the means of linear regression); CF (average consumption figure during 100 h starting 50 after operation start, measured in kg/kg); Conv. (average methanol conversion during 100 h starting 50 after operation start, measured in %); Select. (average formaldehyde selectivity during 100 h starting 50 after operation start, measured in %)

## Claims

1. A fixed bed catalyst comprises a layer A which comprises a catalytic component A1 comprising at least one silver based intermetallic compound composed of the components Ag and M, where M is a metal from the group consisting of Zr and Al.

2. The fixed bed catalyst according to claim 1, wherein the silver based intermetallic compound of the invention is from the group consisting of AgZr, Ag₃Al, and AgZr₂.

3. The fixed bed catalyst according to claim 1 or 2, wherein layer A comprises the catalytic component A1 and at least one additional catalytic component A2 which comprises silver or non-intermetallic alloys thereof.

4. The fixed bed catalyst according to any of claims 1 to 3, wherein the catalytical components of layer A are present in a form selected from the group consisting of net, wads, and granulate.

5. The fixed bed catalyst according to claim 4, wherein the catalytical components of layer A are present in form of granulate.

6. The fixed bed catalyst according to claim 5, wherein the catalytical components of layer A are present in form of granulate having an average particle size preferably in the range from 0.3 mm to 3 mm.

7. The fixed bed catalyst according to any of claims 1 to 6, wherein the fixed bed catalyst comprises an additional layer B which is located downstream to layer A and which comprises a catalytic component B1 consisting of silver based granulate.

8. The fixed bed catalyst according to claim 7, wherein the catalytic component B1 consists of electrolytic silver.

9. The fixed bed catalyst according to claim 7 or 8, wherein the catalytical component B1 is present in form of granulate having an average particle size preferably in the range from 0.5 mm to 5 mm.

10. A method of preparing an activate fixed bed catalyst, wherein the fixed bed catalyst of any of the claims 1 to 9 is exposed to reaction conditions or to activation conditions for at least 1 day, wherein reaction conditions are **characterized by** introduction of the reactant stream, comprising one or more C1-C10 alcohols and one or more other oxidative agents, through the fixed bed catalyst at a temperature in the range from 350°C to 750°C, a gas hourly space velocity in the range from 36,000 h⁻¹ to 1,800,000 h⁻¹ at a gas velocity in the range from 0.1 m s⁻¹ to 15 m s⁻¹, and wherein activation conditions are **characterized by** introduction of a gas mixture, comprising oxygen and/or one or more other oxidative agents and a material capable of being oxidized, through the fixed bed catalyst at a temperature in the range from 350°C to 750°C, a gas hourly space velocity in the range from 36,000 h⁻¹ to 1,800,000 h⁻¹ at a gas velocity in the range from 0.1 m s⁻¹ to 15 m s⁻¹.

11. An activated fixed bed catalyst obtained by the method of claim10.

12. A method of producing C1-C10 aldehydes and/or ketones through the oxidative dehydration of the corresponding C1-C10 alcohols such that the reactant stream, comprising one or more C1-C10 alcohols and one or more oxidative agents at a temperature in the range from 350 to 750°C is led through the fixed bed catalyst of any of the claims 1 to 9 and 11.

13. The method of claim 12, wherein the C1-C10 alcohol consists of methanol, the C1-C10 aldehyde consists of formaldehyde, and the reactant stream is led through the fixed bed catalyst at a temperature in the range from 550 to 750°C.
